# FASCICULE DE BREVET EUROPEEN

(11) **EP 0 519 820 B1**
(45) Date de publication et mention de la délivrance du brevet: **06.12.1995**
(21) Numéro de dépôt: 92401694.2
(22) Date de dépôt: 18.06.1992
(51) Int. Cl.: A61K 31/40, A61K 9/26

(54) **Comprimé matriciel permettant la libération prolongée d'indapamide après administration par voie orale**
Matrixtablette zur verlängerten Indapamidfreisetzung nach oraler Gabe
Matrixtablet for the sustained release of indapamide after oral administration

(30) Priorité: 18.06.1991 FR 9107400
(43) Date de publication de la demande: 23.12.1992
(73) Titulaire: ADIR ET COMPAGNIE, 92415 Courbevoie Cédex (FR)
(72) Inventeur: Cuine, Alain, F-45800 Saint Jean de Braye (FR); Huet de Barochez, Bruno, F-45520 Chevilly (FR); Guez, David, F-92200 Neuilly sur Seine (FR)

(56) Documents cités:
- EP-A- 0 219 161
- WO-A-85/04099
- FR-A- 2 310 764
- FR-A- 2 576 788
- FR-A- 2 588 188
- GB-A- 2 181 053
- US-A- 4 756 911
- US-A- 5 000 962

## Description

La présente invention a pour objet un comprimé matriciel permettant la libération prolongée de l'indapamide, ceci assurant des taux sanguins réguliers et constants après absorption de la forme galénique par voie orale.

L'indapamide, composé de formule (I) : 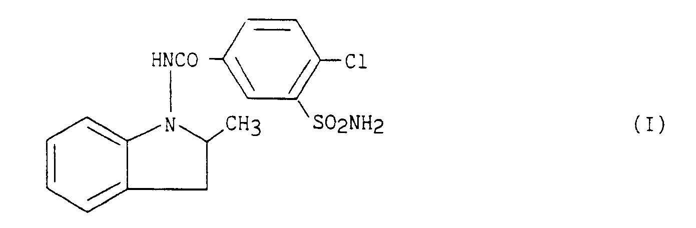
est un dérivé sulfamidique non thiazidique à propriété antihypertensive aux doses habituellement administrées à l'homme.

L'indapamide était jusqu'à présent administré par voie orale à une dose de 2,5 mg par jour au moyen d'une forme à libération immédiate.

Or, une forme à libération immédiate peut conduire, chez certains sujets, à l'obtention de pics sanguins importants. Une forme à libération prolongée permet d'éviter ces pics sanguins et d'obtenir une concentration sanguine régulière chez l'homme. Ceci permet de réduire les effets indésirables pouvant éventuellement survenir par "effet de pic" accompagnés de troubles de type hydroélectrolytiques et métabolites reliés aux variations des taux plasmatiques du principe actif.

Une forme à libération prolongée d'indapamide permet donc d'assurer un meilleur index thérapeutique dans le traitement de l'hypertension artérielle essentielle.

Pour ce faire, il est nécessaire d'assurer une libération prolongée dans le temps, de façon parfaitement contrôlée. La vitesse de libération doit être reproductible et corrélée avec les concentrations sanguines observées après administration.

Parmi les mécanismes pouvant être invoqués pour le contrôle de la diffusion d'un principe actif soluble, on peut en retenir un principal, qui est la diffusion du principe actif au travers d'un gel formé après gonflement d'un polymère hydrophile mis au contact du liquide de dissolution (in vitro), ou du liquide gastro-intestinal (in vivo).

De nombreux polymères ont été décrits comme pouvant permettre la formation de ce gel. Les principaux sont les dérivés de la cellulose, en particulier les éthers de cellulose tels l'hydroxypropylcellulose, l'hydroxyméthylcellulose, la méthylcellulose, la méthylhydroxypropylcellulose, et parmi les différentes qualités commerciales de ces éthers celles présentant des viscosités assez élevées. Il faut noter que les systèmes décrits ne permettent pas théoriquement d'atteindre un ordre zéro dans l'équation de la cinétique de libération.

Les procédés de fabrication couramment utilisés pour la fabrication de tels comprimés matriciels sont soit la compression directe, après mélange des différents excipients et du ou des principes actifs, soit la granulation humide.

Le comprimé matriciel décrit dans la présente invention associe de façon originale deux polymères de familles chimiques différentes permettant d'obtenir une libération du principe actif parfaitement contrôlée. En outre, cette association s'adapte parfaitement aux caractéristiques physico-chimiques de l'indapamide.

Cette libération contrôlée est linéaire pendant plus de huit heures et est telle que 50 pour cent de la quantité totale d'indapamide est libérée entre 5 et 14 heures. D'autre part le comprimé matriciel selon l'invention permet d'obtenir une libération prolongée d'indapamide conduisant à des taux sanguins chez l'homme compris entre 20 et 80 ng/ml, 24 heures au plus après administration du comprimé. La posologie unitaire peut ainsi varier selon l'âge et le poids du patient, la nature et la sévérité de l'affection. D'une manière générale, elle s'échelonne entre 1 et 2,5 mg pour un traitement journalier.

Le premier polymère est un méthylhydroxypropylcellulose de haute viscosité, le second est une polyvidone. L'association de ces deux polymères permet d'obtenir une cinétique de libération in vitro linéaire (d'ordre zéro) sur plus de huit heures. Le pourcentage de polymère dérivé de la cellulose est compris entre 30 et 50 % de la masse totale du comprimé, celui du dérivé de polyvidone est compris entre 2 et 10 % de la masse totale du comprimé.

Ces deux polymères sont mis en oeuvre séparément dans le comprimé pour assurer de façon reproductible le contrôle de la libération du principe actif. Un procédé de fabrication original a lui-même été mis au point afin que chaque polymère utilisé dans la formulation puisse jouer son rôle de la manière la plus efficace possible avec les avantages des deux procédés, granulation humide et compression directe.

Une granulation humide est effectuée avec la polyvidone, afin de créer autour du principe actif un environnement hydrophile favorable à sa bonne dissolution, et également de manière à obtenir un dosage unitaire le plus régulier possible, la teneur en indapamide du comprimé terminé étant d'environ 1 pour cent. Pour cela, un diluant hydrophile, le lactose, est utilisé.

Après cette étape de granulation, un mélange de compression directe est réalisé, puis comprimé.

Les exemples suivants illustrent l'invention mais ne la limitent en aucune façon. La préparation des comprimés à libération prolongée est réalisée selon le procédé de fabrication suivant :
- **Stade A :**: Mélange de l'indapamide, de la polyvidone et du lactose, puis mouillage de ce mélange au moyen d'une solution hydro-alcoolique. La masse humide préparée est ensuite granulée, séchée puis calibrée, de manière à obtenir un granulé dont les caractéristiques physiques permettent un bon remplissage des matrices d'une machine à comprimer rapide.
- **Stade B :**: Mélange du granulé obtenu au stade A avec la méthylhydroxypropylcellulose.
- **Stade C :**: Lubrification du mélange obtenu au stade B avec de la silice colloïdale et du stéarate de magnésium.
- **Stade D :**: Compression du mélange lubrifié obtenu au stade C sur machine à comprimer rotative, de manière à obtenir des comprimés ayant une dureté mesurée par écrasement diamétral d'environ 60 à 75 N.

### EXEMPLE 1 :

Un comprimé à libération prolongée (LP 1) est préparé en utilisant la formule donnée dans le tableau 1, suivant le procédé opératoire dans les stades A à D.

**Tableau 1**

| **Formule unitaire du comprimé LP 1** | |
|---|---|
| Composé | Quantité (mg) |
| Indapamide | 2,5 |
| Lactose | 114,9 |
| Polyvidone | 6,2 |
| Méthylhydroxypropylcellulose | 59,0 |
| Stéarate de magnésium | 2,0 |
| Silice colloïdale | 0,4 |

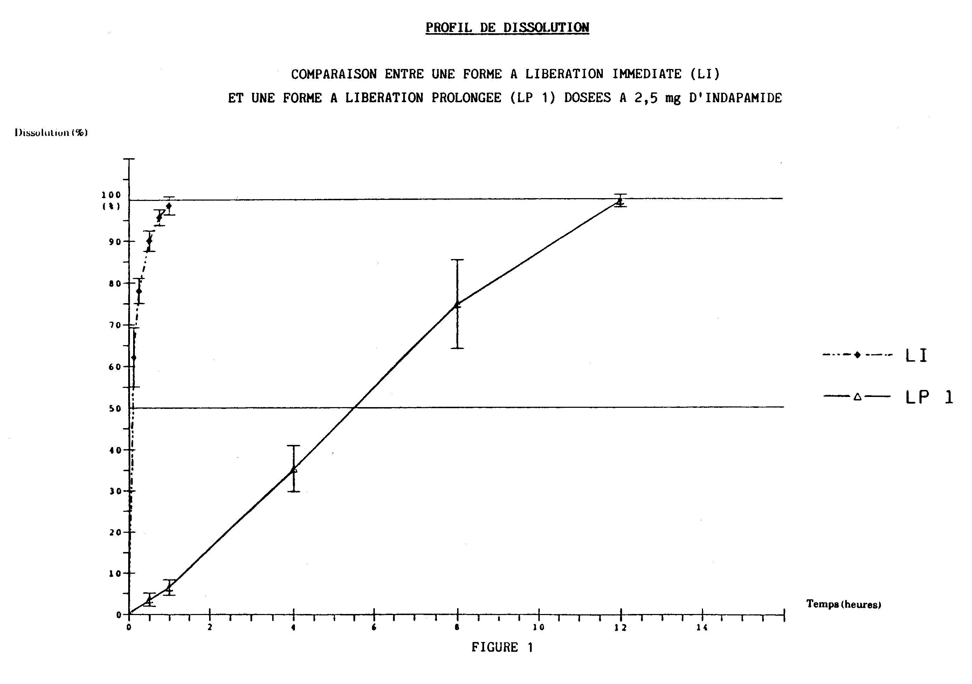

Le profil de dissolution in vitro de cette forme (LP 1) est présenté dans la figure 1 (annexe).

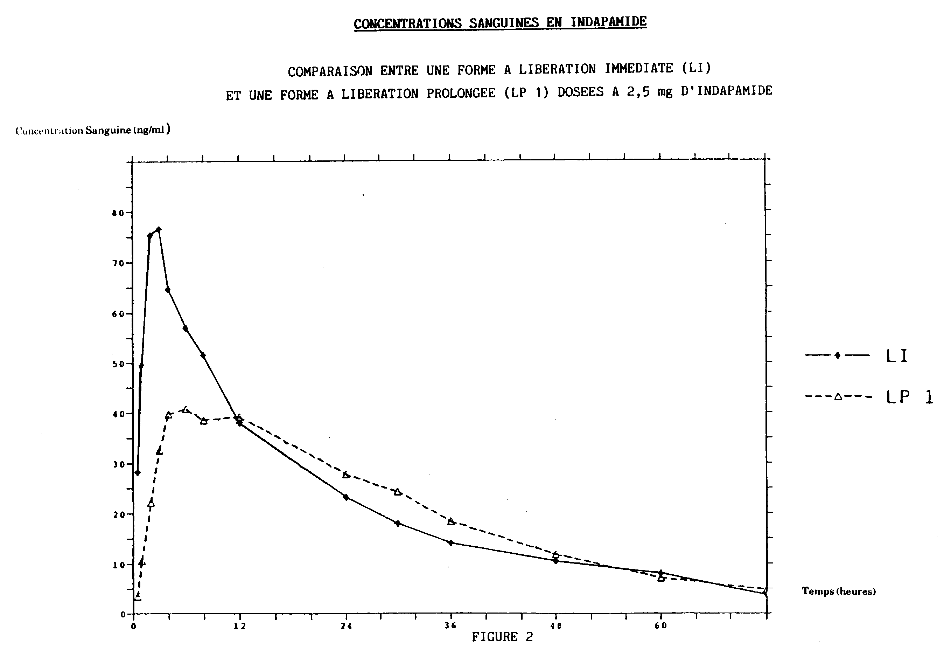

Les concentrations sanguines en indapamide ont été mesurées chez six sujets après administration du comprimé LP 1 ou d'un comprimé à libération immédiate (LI). La courbe moyenne est donnée dans la figure 2 (annexe).

### EXAMPLE 2 :

Un comprimé à libération prolongé (LP 2) est préparé en utilisant la formule donnée dans le tableau 2, suivant le procédé opératoire décrit dans les stades A à D.

**Tableau 2**

| **Formule unitaire du comprimé LP 2** | |
|---|---|
| Composé | Quantité (mg) |
| Indapamide | 2,5 |
| Lactose | 114,9 |
| Polyvidone | 6,2 |
| Méthylhydroxypropylcellusose | 74,0 |
| Stéarate de magnésium | 2,0 |
| Silice colloïdale | 0,4 |

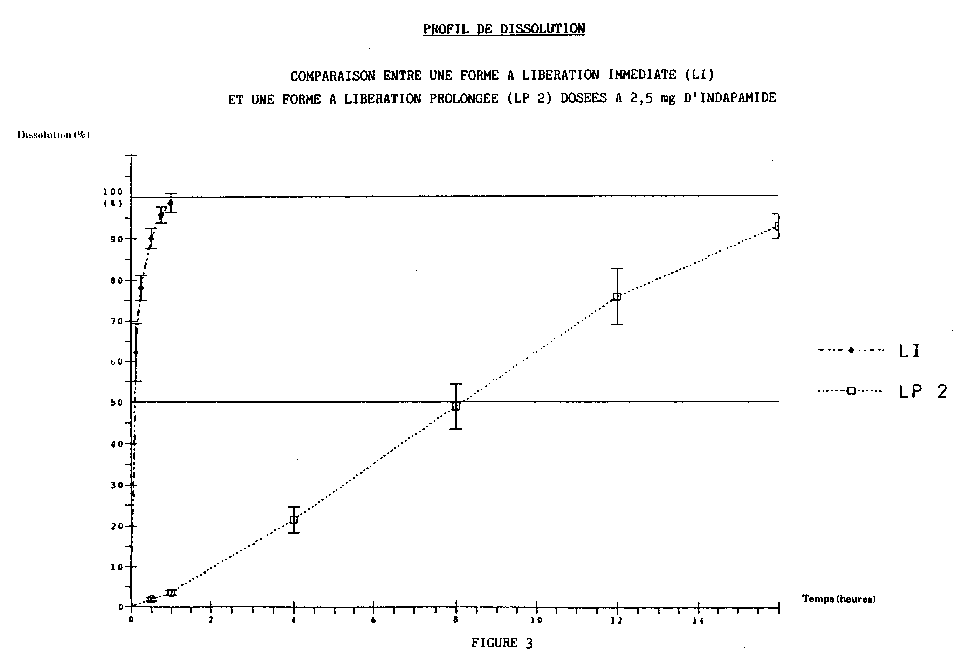

Le profil de dissolution in vitro de cette forme est présenté dans la figure 3 (annexe).

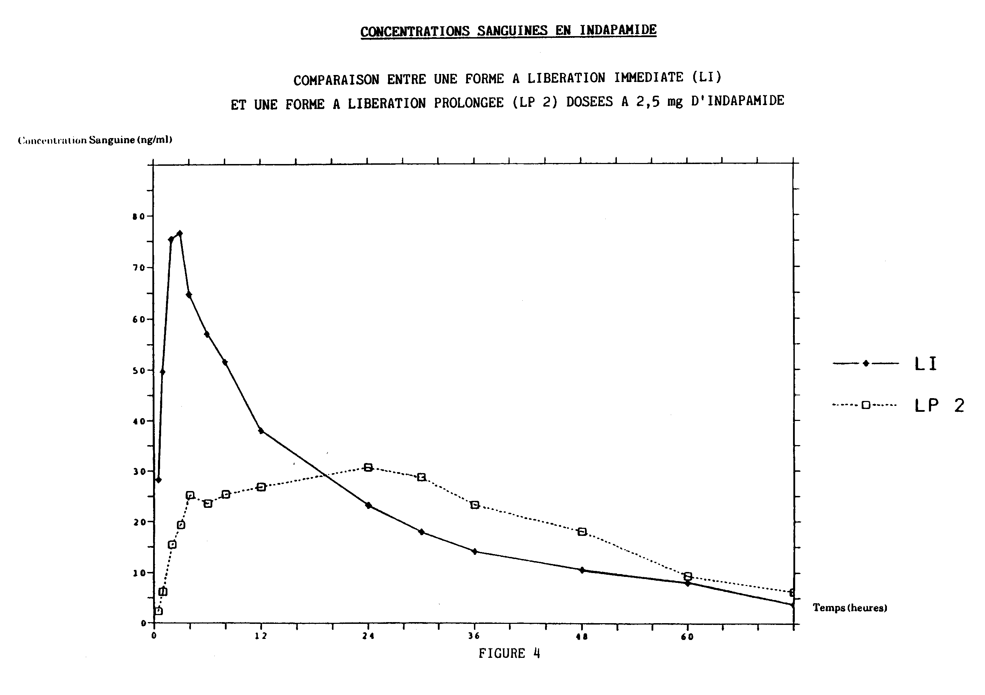

Les concentrations sanguines en indapamide ont été mesurées chez six sujets après administration du comprimé LP 2 ou d'une forme à libération immédiate (LI). La courbe moyenne est donnée dans la figure 4 (annexe).

La comparaison entre les formules décrites dans les exemples 1 et 2 ne différant que par la quantité de méthylhydroxypropylcellulose montre qu'il est aisé de contrôler la cinétique de dissolution in vitro de l'indapamide. La relation avec la cinétique sanguine mesurée in vivo est très bonne. On retrouve deux cinétiques sanguines significativement différentes.

### EXEMPLES 3 à 5 :

Trois comprimés à libération prolongée (LP 3, LP 4 et LP 5) sont préparés en utilisant les formules données dans le tableau 3, suivant le procédé opératoire décrit dans les stades A à D.

**Tableau 3**

| **Formule unitaire des comrimés LP 3, LP 4 et LP 5** | | | |
|---|---|---|---|
| Comprimé | LP 3 | LP 4 | LP 5 |
| | Quantité | | |
| | (mg) | (mg) | (mg) |
| Indapamide | 2,0 | 2,0 | 2,0 |
| Lactose | 115,4 | 113,0 | 113,0 |
| Polyvidone | 6,2 | 8,6 | 8,6 |
| Méthylhydroxypropylcellulose | 74,0 | 74,0 | 59,0 |
| Stéarate de magnésium | 2,0 | 2,0 | 2,0 |
| Silice colloïdale | 0,4 | 0,4 | 0,4 |

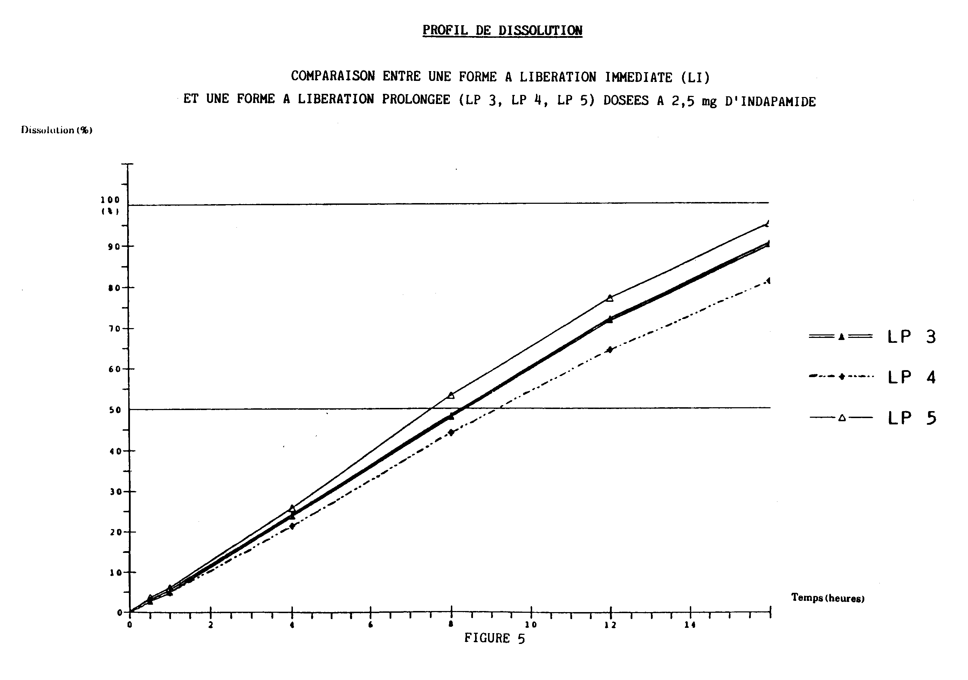

Les profils de dissolution in vitro de ces trois formes sont présentés dans la figure 5 (annexe). Ils montrent qu'en jouant sur la concentration de chaque polymère dans la formule, il est possible de moduler la libération du principe actif.

## Revendications (Revendications pour l'(les) Etat(s) contractant(s) suivant(s): AT, BE, CH, DE, DK, FR, GB, IT, LI, LU, NL, PT, SE)

1. Comprimé matriciel pour la libération prolongée de l'indapamide caractérisé en ce que cette libération prolongée est contrôlée par l'utilisation de méthylhydroxypropylcellulose et de polyvidone, dont les pourcentages sont respectivement compris entre 30 et 50 % et entre 2 et 10 % de la masse totale du comprimé.

2. Comprimé matriciel d'indapamide selon la revendication 1 caractérisé en ce que les pourcentages des dérivés de cellulose et de polyvidone permettent une libération prolongée de l'indapamide linéaire pendant au moins huit heures.

3. Comprimé matriciel d'indapamide selon la revendication 1 caractérisé en ce que les pourcentages des dérivés de cellulose et de polyvidone permettent la libération de 50 pour cent de la quantité totale d'indapamide en un temps compris entre 5 et 14 heures.

4. Comprimé matriciel d'indapamide selon la revendication 1 caractérisé en ce que les pourcentages des dérivés de cellulose et de polyvidone permettent une libération prolongée d'indapamide conduisant à des taux sanguins chez l'homme compris entre 20 et 80 ng/ml, 24 heures au plus après administration du comprimé par voie orale.

5. Procédé de préparation d'un comprimé matriciel d'indapamide selon la revendication 1 caractérisé en ce que l'on utilise à la fois une technique de granulation humide et de compression directe comprenant les étapes suivantes :
Stade (A) : mélange de l'indapamide, de la polyvidone et du lactose, puis mouillage de ce mélange au moyen d'une solution hydro-alcoolique conduisant à une masse humide qui est ensuite granulée, séchée puis calibrée, de manière à obtenir un granulé dont les caractéristiques physiques permettent un bon remplissage des matrices d'une machine à comprimer rapide,
Stade (B) : mélange du granulé obtenu au stade A avec la méthylhydroxypropylcellulose,
Stade (C) : lubrification du mélange obtenu au stade B avec de la silice colloïdale et du stéarate de magnésium,
Stade (D) : compression du mélange lubrifié obtenu au stade C sur machine à comprimer rotative, de manière à obtenir des comprimés ayant une dureté mesurée par écrasement diamétral d'environ 60 à 75 N.

6. Comprimé matriciel d'indapamide selon l'une quelconque des revendications 1 à 4 utile dans le traitement de l'hypertension artérielle essentielle.

## Revendications (Revendications pour l'(les) Etat(s) contractant(s) suivant(s): ES, GR)

1. Procédé de préparation d'un comprimé matriciel pour la libération prolongée de l'indapamide caractérisé en ce que cette libération prolongée est contrôlée par l'utilisation de méthylhydroxypropylcellulose et de polyvidone dont les pourcentages sont respectivement compris entre 30 et 50 % et entre 2 et 10 % de la masse totale du comprimé.

2. Procédé de préparation d'un comprimé matriciel d'indapamide selon la revendication 1 caractérisé en ce que les pourcentages des dérivés de cellulose et de polyvidone permettent une libération prolongée de l'indapamide linéaire pendant au moins huit heures.

3. Procédé de préparation d'un comprimé matriciel d'indapamide selon la revendication 1 caractérisé en ce que les pourcentages des dérivés de cellulose et de polyvidone permettent la libération de 50 pour cent de la quantité totale d'indapamide en un temps compris entre 5 et 14 heures.

4. Procédé de préparation d'un comprimé matriciel d'indapamide selon la revendication 1 caractérisé en ce que les pourcentages des dérivés de cellulose et de polyvidone permettent une libération prolongée d'indapamide conduisant à des taux sanguins chez l'homme compris entre 20 et 80 ng/ml, 24 heures au plus après administration du comprimé par voie orale.

5. Procédé de préparation d'un comprimé matriciel d'indapamide selon la revendication 1 caractérisé en ce que l'on utilise à la fois une technique de granulation humide et de compression directe comprenant les étapes suivantes :
Stade (A) : mélange de l'indapamide, de la polyvidone et du lactose, puis mouillage de ce mélange au moyen d'une solution hydro-alcoolique conduisant à une masse humide qui est ensuite granulée, séchée puis calibrée, de manière à obtenir un granulé dont les caractéristiques physiques permettent un bon remplissage dos matrices d'une machine à comprimer rapide,
Stade (B) : mélange du granulé obtenu au stade A avec la méthylhydroxypropylcellulose,
Stade (C) : lubrification du mélange obtenu au stade B avec de la silice colloïdale et du stéarate de magnésium,
Stade (D) : compression du mélange lubrifié obtenu au stade C sur machine à comprimer rotative, de manière à obtenir des comprimés ayant une dureté mesurée par écrasement diamétral d'environ 60 à 75 N.

6. Procédé de préparation d'un comprimé matriciel d'indapamide selon l'une quelconque des revendications 1 à 4 utile dans le traitement de l'hypertension artérielle essentielle.

## Claims (Claims for the following Contracting State(s): AT, BE, CH, DE, DK, FR, GB, IT, LI, LU, NL, PT, SE)

1. Matrix tablet for the sustained release of indapamide, characterised in that the sustained release is controlled by the use of methylhydroxypropylcellulose and polyvidone, the percentages of which are from 30 to 50% and from 2 to 10%, respectively, of the total mass of the tablet.

2. Indapamide matrix tablet according to claim 1, characterised in that the percentages of the cellulose and polyvidone compounds permit the sustained release of indapamide in a manner that is linear for a period of at least eight hours.

3. Indapamide matrix tablet according to claim 1, characterised in that the percentages of the cellulose and polyvidone compounds permit the release of 50% of the total quantity of indapamide within a period of from 5 to 14 hours.

4. Indapamide matrix tablet according to claim 1, characterised in that the percentages of the cellulose and polyvidone compounds permit the sustained release of indapamide to give blood levels in humans of from 20 to 80 ng/ml, 24 hours at most after administration of the tablet by the oral route.

5. Process for the preparation of an indapamide matrix tablet according to claim 1, characterised in that there are used both a moist granulation technique and a direct compression technique, comprising the following steps:
Step (A): mixing the indapamide, the polyvidone and lactose, and then moistening the mixture with an aqueous-alcoholic solution to yield a moist mass which is then granulated, dried and then graded, so as to obtain a granulate whose physical characteristics allow good filling of the moulds of a rapid compression machine;
Step (B): mixing the granulate obtained in Step A with the methylhydroxypropylcellulose;
Step (C): lubricating the mixture obtained in Step B with colloidal silica and magnesium stearate;
Step (D): compressing the lubricated mixture obtained in Step C in a rotary compression machine, so as to obtain tablets having a hardness, measured by diametral crushing, of approximately from 60 to 75 N.

6. Indapamide matrix tablet according to any one of claims 1 to 4 for use in the treatment of essential arterial hypertension.

## Claims (Claims for the following Contracting State(s): ES, GR)

1. Process for the preparation of a matrix tablet for the sustained release of indapamide, characterised in that the sustained release is controlled by the use of methylhydroxypropylcellulose and polyvidone, the percentages of which are from 30 to 50% and from 2 to 10%, respectively, of the total mass of the tablet.

2. Process for the preparation of an indapamide matrix tablet according to claim 1, characterised in that the percentages of the cellulose and polyvidone compounds permit the sustained release of indapamide in a manner that is linear for a period of at least eight hours.

3. Process for the preparation of an indapamide matrix tablet according to claim 1, characterised in that the percentages of the cellulose and polyvidone compounds permit the release of 50% of the total quantity of indapamide within a period of from 5 to 14 hours.

4. Process for the preparation of an indapamide matrix tablet according to claim 1, characterised in that the percentages of the cellulose and polyvidone compounds permit the sustained release of indapamide to give blood levels in humans of from 20 to 80 ng/ml, 24 hours at most after administration of the tablet by the oral route.

5. Process for the preparation of an indapamide matrix tablet according to claim 1, characterised in that there are used both a moist granulation technique and a direct compression technique, comprising the following steps:
Step (A): mixing the indapamide, the polyvidone and lactose, and then moistening the mixture with an aqueous-alcoholic solution to yield a moist mass which is then granulated, dried and then graded, so as to obtain a granulate whose physical characteristics allow good filling of the moulds of a rapid compression machine;
Step (B): mixing the granulate obtained in Step A with the methylhydroxypropylcellulose;
Step (C): lubricating the mixture obtained in Step B with colloidal silica and magnesium stearate;
Step (D): compressing the lubricated mixture obtained in Step C in a rotary compression machine, so as to obtain tablets having a hardness, measured by diametral crushing, of approximately from 60 to 75 N.

6. Process for the preparation of an indapamide matrix tablet according to any one of claims 1 to 4 for use in the treatment of essential arterial hypertension.

## Patentansprüche (Patentansprüche für folgende(n) Vertragsstaat(en): AT, BE, CH, DE, DK, FR, GB, IT, LI, LU, NL, PT, SE)

1. Matrixtablette für die verzögerte Freisetzung von Indapamid, **dadurch gekennzeichnet,** daß diese verzögerte Freisetzung durch die Verwendung von Methylhydroxypropylcellulose und Polyvidon gesteuert wird, deren Prozentsätze zwischen 30 und 50% bzw. 2 und 10%, bezogen auf die Gesamtmasse der Tablette, liegen.

2. Indapamid-Matrixtablette nach Anspruch 1, **dadurch gekennzeichnet**, daß die Prozentsätze der Cellulosederivate und von Polyvidon eine lineare verzögerte Freisetzung von Indapamid im Verlaufe von mindestens acht Stunden ermöglichen.

3. Indapamid-Matrixtablette nach Anspruch 1, **dadurch gekennzeichnet,** daß die Prozentsätze der Cellulosederivate und von Polyvidon die Freisetzung von 50 % der Gesamtmenge von Indapamid im Verlaufe einer Zeit zwischen 5 und 15 Stunden ermöglichen.

4. Indapamid-Matrixtablette nach Anspruch 1, **dadurch gekennzeichnet,** daß die Prozentsätze der Cellulosederivate und von Polyvidon eine verzögerte Freisetzung von Indapamid ermöglichen, die höchstens 24 Stunden nach der Verabreichung der Tablette auf oralem Wege beim Menschen Blutspiegel zwischen 20 und 80 ng/ml ermöglichen.

5. Verfahren zur Herstellung einer Indapamid-Matrixtablette nach Anspruch 1, **dadurch gekennzeichnet,** daß man gleichzeitig eine Technik der Feuchtgranulation und der Direktverpressung anwendet, welche die folgenden Stufen umfaßt:
Stufe (A): Vermischen von Indapamid, Polyvidon und Lactose, dann Benetzen dieser Mischung mit einer wäßrig-alkoholischen Lösung zur Bildung einer feuchten Masse, die anschließend granuliert, getrocknet und dann kalibriert wird in der Weise, daß man ein Granulat erhält, dessen physikalische Eigenschaften eine gute Füllung der Preßformen einer Schnellpreßvorrichtung ermöglichen,
Stufe (B): Vermischen des in der Stufe A erhaltenen Granulats mit Methylhydroxypropylcellulose,
Stufe (C): Gleitmittelbehandlung der in der Stufe B erhaltenen Mischung mit kolloidalem Siliciumdioxid und Magnesiumstearat,
Stufe (D): Verpressen der in der Stufe D erhaltenen, mit Gleitmittel versehenen Mischung auf einer Rotationspreßvorrichtung in der Weise, daß man Tabletten erhält, die eine durch diametrale Zerstörung gemessene Härte von etwa 60 bis 75 N besitzen.

6. Indapamid-Matrixtablette nach einem der Ansprüche 1 bis 4 zur Behandlung von essentieller Arterienhypertension.

## Patentansprüche (Patentansprüche für folgende(n) Vertragsstaat(en): ES, GR)

1. Verfahren zur Herstellung einer Matrixtablette zur verzögerten Freisetzung von Indapamid, **dadurch gekennzeichnet,** daß diese verzögerte Freisetzung durch die Verwendung von Methylhydroxypropylcellulose und Polyvidon, deren Prozentsätze zwischen 30 und 50 % bzw. 2 und 10 % der Gesamtmasse der Tablette liegen, gesteuert wird.

2. Verfahren zur Herstellung einer Indapamid-Matrixtablette nach Anspruch 1, **dadurch gekennzeichnet,** daß die Prozentsätze der Cellulosederivate und von Polyvidon eine lineare verzögerte Freisetzung von Indapamid im Verlaufe von mindestens acht Stunden ermöglichen.

3. Verfahren zur Herstellung einer Indapamid-Matrixtablette nach Anspruch 1, **dadurch gekennzeichnet,** daß die Prozentsätze der Cellulosederivate und von Polyvidon die Freisetzung von 50 % der Gesamtmenge von Indapamid im Verlaufe einer Zeit zwischen 5 und 15 Stunden ermöglichen.

4. Verfahren zur Herstellung einer Indapamid-Matrixtablette nach Anspruch 1, **dadurch gekennzeichnet,** daß die Prozentsätze der Cellulosederivate und von Polyvidon eine verzögerte Freisetzung von Indapamid ermöglichen, die höchstens 24 Stunden nach der Verabreichung der Tablette auf oralem Wege beim Menschen Blutspiegel zwischen 20 und 80 ng/ml ermöglichen.

5. Verfahren zur Herstellung einer Indapamid-Matrixtablette nach Anspruch 1, **dadurch gekennzeichnet,** daß man gleichzeitig eine Technik der Feuchtgranulation und der Direktverpressung anwendet, welche die folgenden Stufen umfaßt:
Stufe (A): Vermischen von Indapamid, Polyvidon und Lactose, dann Benetzen dieser Mischung mit einer wäßrig-alkoholischen Lösung zur Bildung einer feuchten Masse, die anschließend granuliert, getrocknet und dann kalibriert wird in der Weise, daß man ein Granulat erhält, dessen physikalische Eigenschaften eine gute Füllung der Preßformen einer Schnellpreßvorrichtung ermöglichen,
Stufe (B): Vermischen des in der Stufe A erhaltenen Granulats mit Methylhydroxypropylcellulose,
Stufe (C): Gleitmittelbehandlung der in der Stufe B erhaltenen Mischung mit kolloidalem Siliciumdioxid und Magnesiumstearat.
Stufe (D): Verpressen der in der Stufe D erhaltenen, mit Gleitmittel versehenen Mischung auf einer Rotationspreßvorrichtung in der Weise, daß man Tabletten erhält, die eine durch diametrale Zerstörung gemessene Härte von etwa 60 bis 75 N besitzen.

6. Verfahren zur Herstellung einer Indapamid-Matrixtablette nach einem der Ansprüche 1 bis 4, die für die Behandlung der essentiellen Arterienhypertension geeignet ist.
